# EUROPÄISCHE PATENTANMELDUNG

(11) **EP 1 085 085 A1**
(43) Veröffentlichungstag der Anmeldung: **21.03.2001**
(21) Anmeldenummer: 99118518.2
(22) Anmeldetag: 18.09.1999
(51) Int. Cl.: C12N 5/10, A61K 35/14, A61K 48/00

(54) **Verfahren zur Reduzierung von spezifischen Immunreaktionen**

(71) Anmelder: Sheriff, Ahmed, Dr., 12305 Berlin (DE)
(72) Erfinder: Sheriff, Ahmed, Dr., 12305 Berlin (DE)
(74) Vertreter: Meyers, Hans-Wilhelm, Dr.Dipl.-Chem.

(57) **Zusammenfassung**

Antigen präsentierende Zelle, die überwiegend vorher bestimmte Antigene präsentiert (monoantigene antigen-präsentierende Zelle) dadurch gekennzeichnet, dass in der monoantigenen antigen-präsentierenden Zelle eine der Funktionen co-stimulatorischer Rezeptoren, wie ein B7 - und/oder CD40-Rezeptor supprimiert ist.

## Beschreibung

Gegenstand der vorliegenden Erfindung sind Antigen präsentierende Zellen, Verfahren zur Herstellung Antigen präsentierender Zellen, Arzneimittel enthaltend Antigen präsentierende Zellen sowie Verwendung der Antigen präsentierenden Zellen .

### Beschreibung

Seit knapp 10 Jahren werden für verschiedene Krankheiten und in Tiermodellen gentherapeutische Verfahren entwickelt und angewendet. Bisher sind sie noch nicht in die Routine überführt. Meist handelt es sich dabei um die Behandlung von genetisch bedingten, schweren Erkrankungen, für die andere Therapien nicht zur Verfügung stehen. Weiterhin werden Gentherapien zur Behandlung von schweren und ebenfalls nicht therapierbaren Krebserkrankungen eingesetzt.

Wenig Aufinerksamkeit hat die experimentelle Medizin bisher der Behandlung von Allergien und Autoimmunerkrankungen mittels gentechnischer Methoden geschenkt.

### Allgemeines zu Allergien

Allergien verursachen beträchtliche Kosten im Gesundheitswesen der Industrieländer. Immerhin sind schätzungsweise mindestens 20% der Bevölkerung gegen irgendeine Substanz allergisch. Die meisten Betroffenen leiden an allergischer Rhinitis, zu der insbesondere der Heuschnupfen zählt, oder an Bronchialasthma: Sie niesen oder ringen nach Luft, nachdem sie bestimmte Pollen oder andere im allgemeinen harmlose Substanzen eingeatmet haben. Viele Kinder und einige Erwachsene reagieren auch allergisch auf Nahrungsmittel. Andere erleiden Hautausschläge oder sogar einen allergischen Schock, nachdem sie Medikamente wie Penizillin erhalten haben. Bei wieder anderen rufen Bienenstiche starke lokale Schwellungen oder schwere systemische - den gesamten Organismus erfassende - Störungen hervor. Im Extremfall können allergische Anfälle sogar zum Tod führen. Allein die unmittelbare medizinische Versorgung von Asthmapatienten hat in den USA 1990 schätzungsweise 3,6 Milliarden Dollar verschlungen und damit rund ein Prozent aller Kosten im Gesundheitswesen ausgemacht.

Mittlerweile ist bekannt, dass einige der zellulären und molekularen Wechselwirkungen bei allergischen Reaktionen oft ähnlich ablaufen, unabhängig davon, auf welche Substanzen der einzelne anspricht und welche Symptome er entwickelt. Gewisse Begleiterscheinungen der Allergien treten normalerweise ausschließlich auf, wenn das Immunsystem Parasiten bekämpft. So reagiert der Körper auf Schmarotzer ebenso wie auf Allergene mit der massiven Produktion von Molekülen, die als Immunglobulin-E-Antikörper (IgE) bezeichnet werden. Die Produktion dieser Antikörper wird durch T-Helfer-Zellen induziert, die wiederum von antigen-präsentierenden Zellen aktiviert werden.

### Sensibilisierung

Unterschiedliche Allergene rufen unter anderem deswegen verschiedenartige Symptome hervor, weil sie mit dem Immunsystem in verschiedenen Körperregionen in Berührung kommen. In den oberen Luftwegen erzeugt die fehlgeleitete Immunreaktion Niesen und eine verstopfte Nase. In den unteren Luftwegen können dagegen die Bronchien sich verengen und verschleimen, so dass typische asthmatische Symptome auftreten. Entsprechend rufen Immunaktivitäten in den Geweben des Magen-Darm-Traktes Übelkeit, Bauchkrämpfe, Durchfall oder Erbrechen hervor.

Schließlich vermag ein Allergen, dass auf irgendeinem Weg ins Blut gelangt, eine Anaphylaxe auszulösen: eine allergische Reaktion in weit von seiner Eintrittsstelle entfernten Körperregionen. Schwere anaphylaktische Schocks können alle normalen Körperfunktionen durcheinanderbringen und tödlich enden.

Auch wenn sich die allergischen Reaktionen unterschiedlich äußern, werden sie doch stets durch den gleichen Mechanismus in Gang gesetzt: die Sensibilisierung. Dazu kann bereits der einmalige Kontakt mit einem Allergen, typischerweise einem Eiweißstoff, genügen. In den Luftwegen oder anderen Geweben trifft die allergieauslösende Substanz auf sogenannte Fresszellen oder Makrophagen. Diese verschlingen den Fremdstoff, zerstückeln ihn und präsentieren die Fragmente auf der Zelloberfläche mit MHC II-Molekülen. Im weiteren Verlauf erkennen einige T-Helfer-Lymphozyten die dargebotenen Bruchstücke und binden daran. Die T-Helfer-Lymphozyten werden von den Makrophagen aktiviert und aktivieren dann ihrerseits einige B-Lymphozyten, die gleichfalls das Allergen erkennen. Die B-Zellen reifen dann zu Antikörper produzierenden Plasmazellen aus. Zunächst sind dies Antikörper vom sogenannten IgM-Typ; ab einem bestimmten Zeitpunkt schalten die Plasmazellen jedoch auf IgE-Antikörper um.

Bis die Antikörper hergestellt sind, können Tage oder Wochen vergehen, und das Allergen, welches ihre Produktion in Gang gesetzt hat, ist dann womöglich schon lange verschwunden. Nicht so die IgE-Moleküle. Mit ihrer Fc-Region heften sie sich an IgE-Rezeptoren zweier unterschiedlicher Klassen von Immunsystemzellen. Bei der einen handelt es sich um Mastzellen, die sich im Körpergewebe für gewöhnlich in der Nähe von Blutgefäßen und Epithelzellen ansiedeln. Über das Epithel besteht Kontakt mit der Außenwelt (darunter fällt auch das Epithel der Atemwege und des Magen-Darm-Traktes). IgE-Antikörper binden außerdem an basophile Granulozyten (Basophile). Diese Zellen zirkulieren allerdings im Blutstrom.

Hat die Produktion der IgEs einmal begonnen, hält sie offenbar Monate, ja manchmal sogar Jahre an. Folglich besetzen sie unablässig IgE-Rezeptoren auf Mastzellen und Basophilen - bereit beim nächsten Allergenkontakt augenblicklich in Aktion zu treten.

### Akute Symptome

Während also die erste Begegnung mit einem Allergen selbst bei Personen, die sich später als Allergiker entpuppen, keine Symptome hervorruft, leitet die Zweitexposition ein Stadium der Überempfindlichkeitsreaktion ein, welches auch äußerlich in Erscheinung tritt. Innerhalb von Sekunden nach dem Kontakt mit menschlichem Gewebe bindet der allergieauslösende Stoff an die IgEs der Mastzellen. Heftet er sich dabei an zwei oder mehr IgE-Moleküle zugleich, bildet er eine Brücke zwischen ihnen. Solche Quervernetzungen lassen die betroffenen IgE-Rezeptoren dichter zusammenrücken, und dies aktiviert die Zelle, so dass sie hochwirksame Substanzen ausschüttet, die auf direktem Wege allergische Symptome erzeugen. (Die Freisetzung kann auch auf andere Arten hervorgerufen werden; von allergischen Reaktionen spricht man nur, wenn IgEs beteiligt sind). Die wichtigste dieser Substanzen ist Histamin. Es kann sowohl die Schleimbildung in den Epithelien anregen und so zur Verstopfung der Luftwege beitragen als auch die glatte Muskulatur, die wie ein elastisches Band Bronchien und Därme umschlingt, kontrahieren lassen. Ferner vermag es die feinen Blutgefäße zu weiten und durchlässiger zu machen, so dass Flüssigkeit ins Gewebe sickern kann. Rötungen und Schwellungen sind die Folge. Betreffen diese Gefäßveränderungen große Teile des Körpers, können sie ein tödliches Kreislaufversagen auslösen: Bei einem solchen Schock fällt der Blutdruck jäh so stark ab, dass die Sauerstoffversorgung von Herz und Gehirn nicht mehr gewährleistet ist.

Die zweite Gruppe von Mediatoren besteht hauptsächlich aus Prostaglandinen und Leukotrienen. Sie werden erst erzeugt, nachdem die Allergenmoleküle sich an die IgEs auf den Zellen angelagert haben. Wie Histamin verengen sie die Bronchien und erweitern die Blutgefäße. Ihre Wirkung hält allerdings länger an.

Zusätzlich stoßen stimulierte Mastzellen eine Vielzahl potentiell toxischer Enzyme aus. Offenbar setzen sie ferner Cytokine frei, die die Aktivitäten anderer Immunzellen regulieren.

### Behandlung: 1. Die allergische Rhinitis

Antihistaminika erweisen sich in der Regel als wirksam und dienen immer noch als Standardtherapie. Die neuesten Varianten können die Blut-Hirn-Schranke nicht mehr ohne weiteres passieren und machen die Patienten nicht mehr müde. Wenn bei einer schweren Entzündung Antihistaminika wirkungslos bleiben, helfen oft inhalierbare Corticosteroide, die gewöhnlich zur Linderung der chronischen Entzündung bei Asthma verschrieben werden.

In schweren Fällen kann die schon im Jahre 1911 eingeführte Immuntherapie oder Hyposensibilisierung (auch als Allergiespritzen oder Desensibilisierung bekannt) auf lange Sicht Erleichterung verschaffen. Bei dieser Behandlung injizieren Ärzte den Patienten steigende Dosen des Allergens, auf das diese empfindlich reagieren. In allen Fällen ist die Dosis ausschlaggebend: Zu wenig Allergen verleiht keine Toleranz. Außerdem ist der Schutz selten vollständig.

### 2. Asthma

Bronchodilatatoren sind die meistverwendeten Arzneimittel bei Asthma. Sie lindern die durch Histamin und andere Bronchokonstriktoren hervorgerufenen Symptome sehr schnell, beeinflussen die zugrundeliegende Entzündung jedoch wahrscheinlich nicht. Außerdem kann ihr übermäßiger Gebrauch eine Gegenreaktion des Körpers hervorrufen, so dass nach Abklingen ihrer Wirkung der Atemstrom stärker behindert ist als zuvor. Zusätzlich kommen die unter 1. aufgezählten Methoden zur Anwendung.

### 3. Anaphylaktische Reaktionen

Insektenstiche rufen bei manchen Menschen Anaphylaxien aus. In schweren Fällen führen diese zum Tod durch z.B. Kreislaufversagen oder Ersticken. Jede schwere Anaphylaxie - gleich ob sie zum ersten oder fünfzehnten Mal auftritt - ist ein Notfall, bei dem zunächst versucht werden muss, die bedrohlichsten Symptome zu beherrschen. Meist geschieht das durch Injektion von Adrenalin, welches die Freisetzung der Mediatoren hemmt, die Luftwege öffnet und der Erweiterung der Blutgefäße entgegenwirkt. Man kann vorbeugend durch eine Immunisierung mit dem Gift des gesundheitsbedrohenden Insekts agieren.

### Neue Ansätze

In der Erprobung im Tiermodell befindet sich die auf DNA basierende Immunisierung mit einem Allergen (Der p 5) der Milbe *Dermatophagoides pteronyssinus*. Diese Immunisierung resultiert in einer Produktion von IgG, aber nicht IgE, und resultiert in einer 90%tigen Reduktion der Mengen an spezifischem IgE, welche durch klassische Sensibilisierung mit Der p 5 und Alaun als Adjuvant oder allergeninduzierte Rhinitis hervorgerufen wurden.

Eine weitere neue Strategie ist die Verwendung von humanisierten monoklonalen Anti-IgE-Antikörpern gegen die FcεRI-Binderegion für IgE. Dadurch wird die Bindung von IgE an den IgE-Rezeptor verhindert, so dass keine Mediatoren der allergischen Reaktion von Mastzellen oder Basophilen ausgeschüttet werden können.

Diese Strategie hat in klinischen Studien bei Patienten mit allergischer Rhinitis und allergischem Asthma gezeigt, dass diese Antikörper gut toleriert werden und die allergischen Reaktionen reduzieren.

(siehe auch L.M. Lichtenstein: "Allergie und Immunsystem"; in Spektrum der Wissenschaft Spezial: Das Immunsystem; 1994; 74-83; S-K Huang, K-Y Chua und K-H Hsieh: Allergen gene transfer. Current Opinion in Immunology 1997; 800-804; C. Heusser und P. Jardieu: Therapeutic potential of anti-IgE antibodies. Current Opinion in Immunology 1997; 805-814).

### Allgemeines zu Transplantationen

Die Transplantation von Geweben, um kranke Organe zu ersetzen, ist heute eine wichtige medizinische Therapie. In den meisten Fällen stellt eine Reaktion des anpassungsfähigen Immunsystems gegen das Transplantat die größte Bedrohung für eine erfolgreiche Behandlung dar. Reaktionen des anpassungsfähigen Immunsystems werden von Antigen-präsentierenden Zellen durch Aktivierung von T-Helfer-Lymphozyten induziert. Bei der Transfusionen von Blut, welches das erste und am häufigsten verwendete Transplantat ist, müssen die ABO und Rh Blutgruppenantigene abgeglichen werden, damit die schnelle Zerstörung unpassender Erythrozyten vermieden wird. Bei anderen Geweben müssen die sehr polymorphen Haupthistokompatibilätskomplexe (MHC) aufeinander abgeglichen werden, da diese fast immer die Immunreaktion auslösen. Leider ist der perfekte Abgleich der MHCs außer bei Verwandten fast unmöglich.

Obwohl die Immunreaktion Organtransplantationen schwierig macht, gibt es wenige Alternativen bei Organausfällen. Die Verwendung von potenten immunsuppressiven Drogen, besonders Cyclosporin A und FK-506, die die Aktivierung von T-Zellen verhindern, macht Organtransplantationen erfolgreich. Trotzdem laufen hier einige Probleme auf, da das Leiden, welches das eigene Organ zerstört hat, auch das fremde Organ zerstört. Außerdem steigt durch die Unterdrückung des Immunsystems, das Risiko an Krebs oder Infektionen zu erkranken. Zudem ist die Prozedur sehr kostspielig (Janeway and Travers 1997).

### Allgemeines zu Autoimmunerkrankungen

Autoimmunerkrankungen gehören zu den chronischen Erkrankungen. Zu ihnen zählen Rheuma in verschiedensten klinischen Ausprägungen, Diabetes, Multiple Sklerose, bestimmte Formen von Herzmuskelentzündungen und von Schilddrüsenerkrankungen. Die Reihe von Autoimmunerkrankungen ließe sich beliebig fortsetzen, wobei ca. 90% allerdings epidemiologisch nur einen kleinen Prozentsatz ausmachen.

Die klinischen Verläufe von Autoimmunerkrankungen selbst bei ein- und derselben Diagnose können sehr unterschiedlich sein. Z. T. werden schubartige Krankheitsverläufe beobachtet. Entsprechend werden die Behandlungen vom Arzt individuell gestaltet.

Einige der Autoimmunerkrankungen sind antikörpervermittelt. Darunter wird in der Immunologie verstanden, dass der Organismus aus meist nicht bekannten Ursachen Antikörper produziert, welche sich gegen körpereigene zelluläre Strukturen (Autoantigene) richten. Sind diese Antikörper einmal gebildet, lösen sie durch ihre Interaktion und Bindung an die jeweiligen organ- oder zellspezifischen Strukturen eine zell- und gewebszerstörende Reaktion des Immunsystems aus. Letztendlich führt diese dann zu dem klinischen Bild der Erkrankung (Steinman 1994).

Beispiel hierfür ist die Schilddrüsenerkrankung Morbus Basedow, aber auch eine Form der Herzmuskelentzündung, die zur Dilatativen Cardiomyopathie führt. In einigen Fällen sind sowohl die Targets, gegen die sich die Autoantikörper richten, als auch die Autoantikörper selbst, genau charakterisiert.

WO-A-96/18736 offenbart ein Verfahren und ein Reagenz zur Behandlung von arthritischen Bedingungen, Induktion von Toleranz bei Verpflanzungen und Umkehrung von Immunreaktionen. Dabei wird die Möglichkeit, die Expression von B7-1, B7-2 und B7-3 durch Ribozyme oder Antisense-RNA zu unterdrücken, theoretisch erörtert. Die Unterdrückung der B7-Mengen durch ein B7-Ribozym und B7-Antisense-RNA werden zwar nicht durch Experimente verifiziert, aber eine reine Unterdrückung von B7 würde im besten Falle eine allgemeine Reduzierung der gesamten Reaktionen des anpassungsfähigen Immunsystems bewirken.

Die WO-A-95/32734 beschreibt die Verhinderung co-stimulatorischer Aktivitäten von antigen-präsentierenden Zellen, indem FcyRII-Rezeptoren durch aggregierte IgG-Moleküle vernetzt werden. Eine Unterdrückung von T-Zell-vermittelten Krankheiten soll erreicht werden, indem gleichzeitig die FcyRII-Rezeptoren vernetzt und spezifische Antigene verabreicht werden.

Die WO-A-98/29124 betrifft injizierte Antisense-Oligonukleotide, mit denen die B7-Mengen reduziert werden sollen. Damit kann die Expression von B7 höchstens unspezifisch in allen Zellen behindert werden.

WO-A-97/44450 offenbart die Erhöhung oder Unterdrückung von Prozessen, die auf sequenzspezifischen Interaktionen von Nukleinsäuren mit der Zellmaschinerie beruhen. Insbesondere können Genpromotor-unterdrückende Nukleinsäuren eingesetzt werden, die mit Genpromotormotiven für die Erhöhung oder Unterdrückung der Transkription eines Zielgens interagieren. Im Detail werden mit einer Utron-Nukleinsäure, dem TSU, die Expression von MHCs der Klassen I und II, sowie von ICAM-1, B7-1, B7-2 und FcyR behindert. Ein solcher Eingriff könnte insbesondere für Organtransplantate von Nutzen sein, bei denen die Expression von MHC-Molekülen die entscheidenden Abstoßungsreaktionen hervorruft. Utron-Nukleinsäuren stammen aus den 3'-untranslatierten Regionen von mRNAs. Diese 3'-untranslatierten Regionen werden so genannt, wenn sie *in vivo* Aktivitäten entfalten, die zelluläre Prozesse durch sequenzspezifische Interaktionen mit einem Teil der zellulären Maschinerie stimulieren oder inhibieren. Bei der zellulären Maschinerie handelt es sich gemäß WO -A-97/44450 um Promotoren. Es wird dort zwar über die Möglichkeit der Unterdrückung der B7-Expression (1 und 2) mit gentechnischen Methoden spekuliert, aber eine solche Inhibition kann mit dem aufgezeigten Mittel nur in Zusammenhang mit der Inhibition weiterer Gene (MHCs der Klassen I und II, sowie von ICAM-1 und FcyR) geschehen. Eine solch weitreichende Inhibition ist kontraproduktiv, da man auf eine hohe MHC-Expression angewiesen ist. Außerdem ist eine Regulation auf der Promotorebene nachteilig, da keine Möglichkeit besteht, um hinreichend spezifisch zu wirken.

Das der Erfindung zu Grunde liegende Problem besteht unter anderem darin, gentechnologische, therapeutisch nutzbare Produkte für die Reduktion von spezifischen Immunreaktionen zur Verfügung zustellen, bei denen Targets (Antigene, Autoantigene) und Antikörper, Autoantikörper bekannt sind.

Gelöst wird das Problem durch die erfindungsgemäße antigen-präsentierende Zelle, die überwiegend vorher bestimmte Antigene präsentiert (monoantigene antigen-präsentierende Zelle) und dadurch gekennzeichnet ist, dass in der monoantigenen antigen-präsentierende Zelle eine der Funktionen co-stimulatorischer Rezeptoren, wie ein B7 - und/oder CD40-Rezeptor supprimiert ist.

Figur 1 zeigt ein Schema zur Funktion der Genmanipulation auf zellulärer Ebene.
a) normaler Mechanismus der T-Helfer-Zell-Aktivierung über Monozyten
b) Mechanismus der Monozyten nach Genmanipulation. Ausbleiben der T-Helfer-Zell-Aktivierung nach Genmanipulation der Monozyten.

Figur 2 zeigt eine "Normale" Immunreaktion zur Antikörperbildung, Antigen-präsentierende Monozyten als Induktoren der Antikörperproduktion. Monozyten nehmen als fremd erkannte Moleküle auf und bringen sie auf die Zelloberfläche (Antigenpräsentation). Gemeinsam mit einem Oberflächenmolekül (B7) wird das Antigen den T-Helferzellen präsentiert. Das präsentierte Antigen wird von T-Helfer-Lymphozyten erkannt, die ihrerseits B-Zellen zur Antikörperproduktion anregen. Das Schema ist stark vereinfacht.

Figur 3 zeigt schematisch die Funktion der Genmanipulation auf molekularer Ebene: der Co-stimulierende Rezeptor B7 wird inaktiviert.

Figur 4 (I) zeigt die Funktion der Genmanipulation auf molekularer Ebene: das Autoantigen wird von den B7-inhibierten antigenpräsentierenden Zellen synthetisiert

Figur 4 (II) zeigt einen Vergleich der Antigenpräsentation von "normalen" und gentechnisch veränderten Monozyten.

Figur 5 demonstriert die Funktion der genetisch veränderten Monozyten im Körper.

Vorzugsweise ist die erfindungsgemäße monoantigene antigen-präsentierende Zelle ein Monozyt, eine dendritische Zelle und/oder ein Makrophage.

Die APCs sind die Schaltstellen des anpassungsfähigen Immunsystems. Nur sie können eine solche Immunantwort auslösen. Dies sei in folgenden Abbildungen am Beispiel von Monozyten und der durch T-Helfer-Zellen ausgelösten Antikörperproduktion gezeigt:

Innerhalb des Immunsystems spielen die Antikörper normalerweise als Abwehrmoleküle ("humorale Immunreaktion") eine wichtige Rolle. Sie werden von ausgereiften B-Lymphozyten produziert. Die Induktion und Produktion der Antikörper erfolgt allerdings nicht unabhängig von den restlichen Zellen des Immunsystems; vielmehr wird diese humorale Immunreaktion von anderen Zellen des Immunsystems gesteuert.

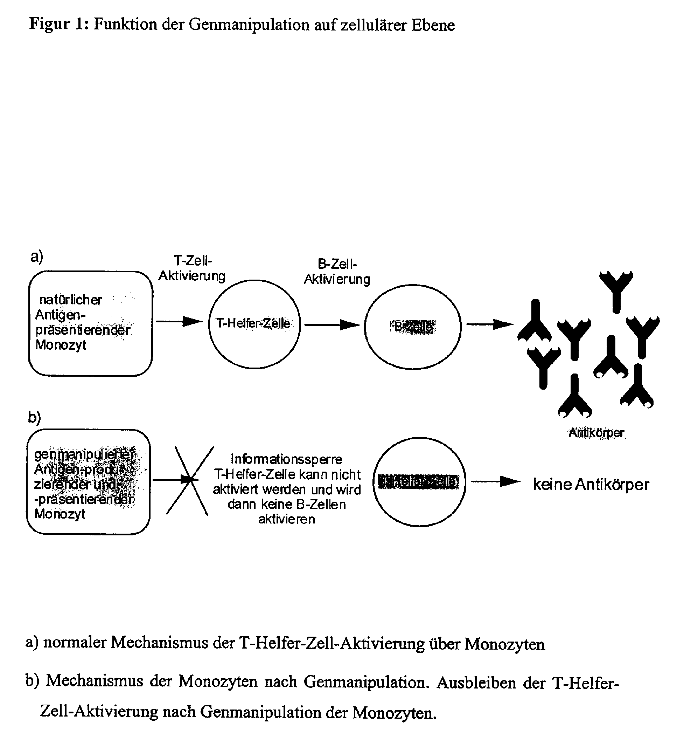

Die Antikörperproduktion - und so auch die Produktion der pathologischen Autoantikörper - lässt sich nicht aufrechterhalten ohne die Hilfe und Vermittlung von Monozyten und T-Helfer-Lymphozyten, die ihrerseits antigenspezifisch die B-Lymphozyten aktivieren (Fig. 1).

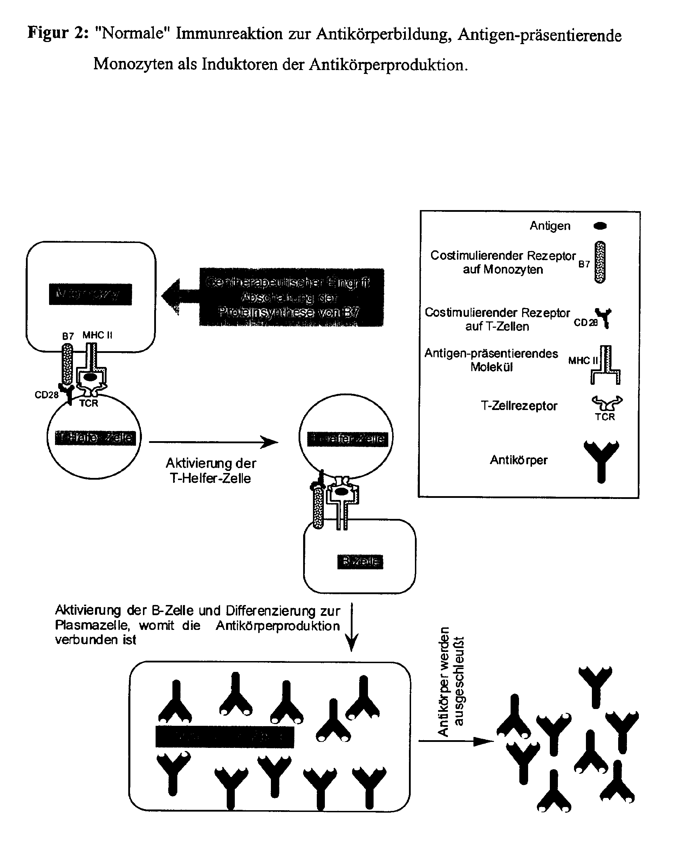

Die molekularen Mechanismen der Interaktion - des Zell-Zell-Kontakts - von Monozyten mit den T-Helfer-Lymphozyten ist auf Rezeptorebene bis ins Detail bekannt (Figur 2).

Handelt es sich bei dem Antigen z.B. um ein Bakterienprotein, ist die Immunreaktion für den Organismus nützlich. Ist das Antigen allerdings eine körpereigene Struktur, spricht man von einer (pathologischen) Autoimmunreaktion.

Neben dem zu präsentierenden Antigen (gegen welches sich die zu produzierenden Antikörper richten) sind auf der Zelloberfläche verschiedene Rezeptoren und Hilfsrezeptoren am Zell-Zell-Kontakt und der Zellaktivierung beteiligt. Ein essentielles Molekül der interzellulären Wechselwirkung bei der Antigenpräsentation (Kontakt von Monozyt mit den T-Helfer-Lymphozyten) ist ein costimulierender Rezeptor mit der Bezeichnung B7 (Figur 2)

Die erfindungsgemäße monoantigene antigen-präsentierende Zelle weist vorzugsweise durch eine Transformation einer antigen-präsentierenden Zelle mit nukleinsäurehaltigem Material eine erhöhte Expression eines Antigens auf, wobei die antigen-präsentierende Zelle im wesentlichen nur eine Sorte eines Antigens präsentiert.

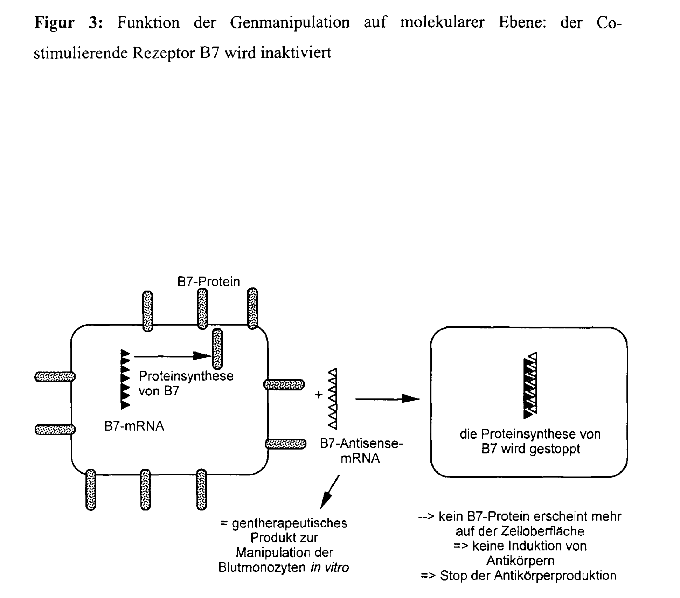

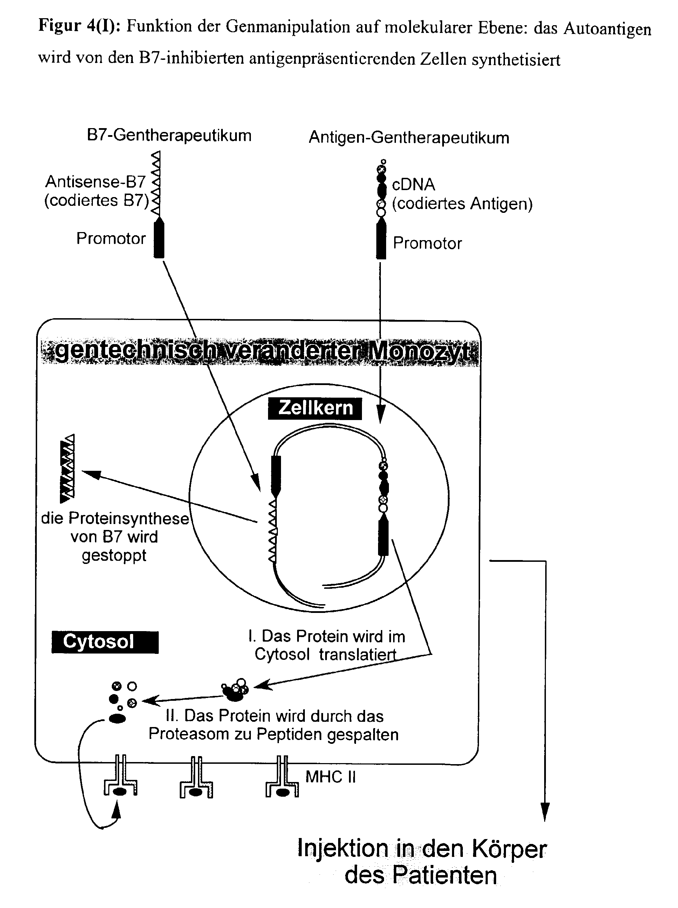

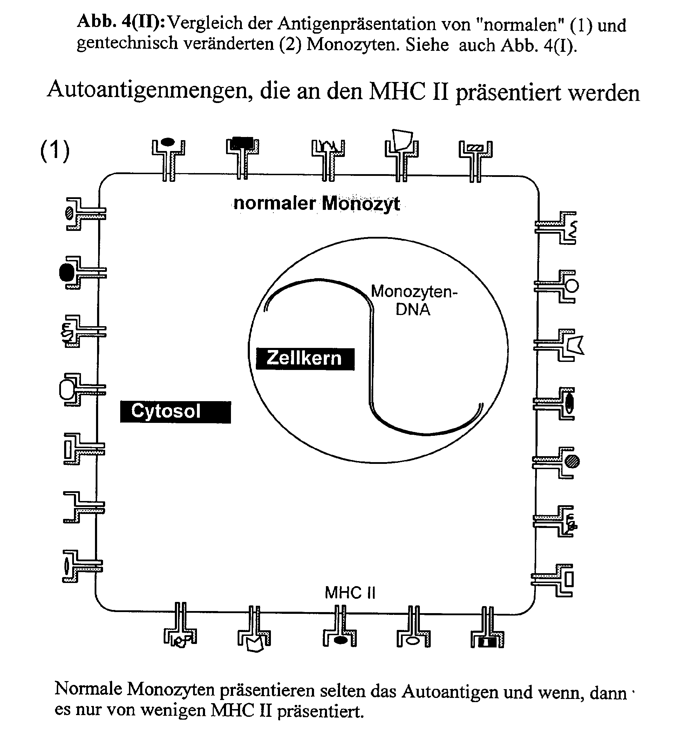

Das konzipierte gentherapeutische Verfahren beruht auf zwei parallelen gentechnologischen Eingriffen an patienteneigenen Blutmonozyten. Die Eingriffe erfolgen mittels geeigneter Sonden und bewirken die Verminderung von B7-Molekülen durch die Behinderung und damit der Verhinderung der Ausbildung des Moleküls auf der Oberfläche der Monozyten (Figur 3) und gleichzeitig eine starke Präsentation des Autoantigens (Figur 4).

### Verminderung der spezifischen Antikörperproduktion durch Gentherapie

Die Produktion pathologischer Autoantikörper wird durch die Manipulation von Monozyten spezifisch abgeschaltet. Der Co-Rezeptor B7, ohne den die Antigenpräsentation bzw. die Induktionskaskade zur Antikörperproduktion nicht anläuft, wird unterdrückt. Hinter dem Begriff B7 verbergen sich zwei unterschiedliche Co-Rezeptoren, die als CD80 (B7-1) und CD86 (B7-2) bezeichnet werden. Ihre Strukturen sind bekannt.

Nach dem Abschalten der Antikörperproduktion verschwinden die im Blut zirkulierenden Autoantikörper aufgrund des natürlichen Abbaus und des fehlenden Nachschubs.

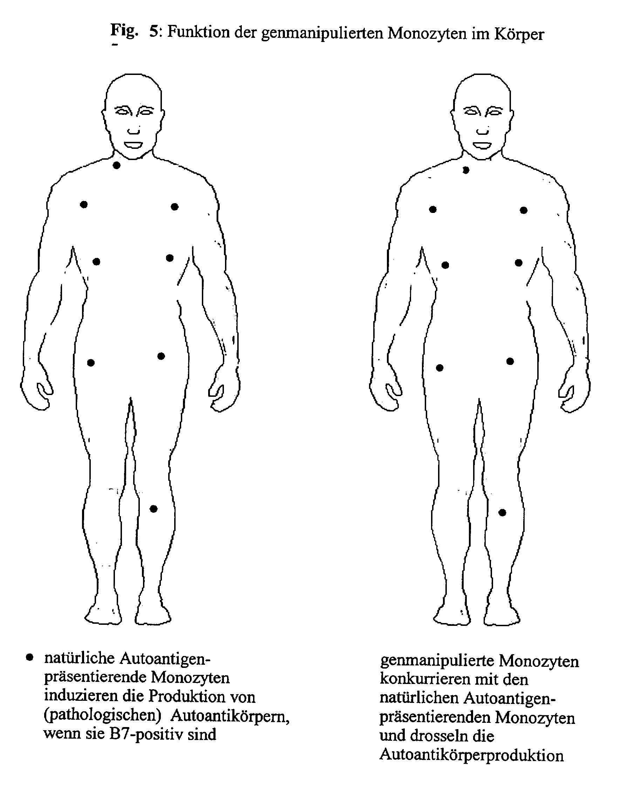

Nach der *in vitro* Manipulation der Monozyten werden die Zellen in die Blutbahn des Patienten zurückgegeben. Die genmanipulierten Monozyten schalten nunmehr die im Organismus befindlichen entsprechenden T-Helfer-Lymphozyten ab. Die genmanipulierten Zellen treten dabei unmittelbar in Konkurrenz zu den bereits im Organismus (vorzugsweise Blutbahn und Lymphsystem) vorhandenen Autoantigen-präsentierenden Monozyten, die allerdings B7 auf der Zelloberfläche tragen und normalerweise die T-Helfer-Lymphozyten und damit die Antikörperproduktion aktivieren (Figur 5).

### Antigenpräsentation bei gentechnisch veränderten Monozyten

Die cDNA eines Proteins, das als Autoantigen die Produktion pathologischer Autoantikörper hervoruft, wird in das Monozytengenom integriert. Diese Erbinformation dient dann zur Überproduktion des Autoantigens. Peptide dieses Autoantigens werden daraufhin bevorzugt an MHC II und/oder MHC I präsentiert (siehe auch Figur 4(II)). MHC II präsentiert die Peptide den T-Helferzellen und versucht solche zu finden, die spezifisch die präsentierten Proteine erkennen. Wenn der Co-Rezeptor B7 nicht gleichzeitig an der Zelloberfläche erscheint, werden die T-Helferzellen stillgestellt und unterliegen einem vorzeitigen Zelltod.

Alle genmanipulierten Monozyten präsentieren an den meisten ihrer MHC II-Komplexe das Autoantigen, während *in vivo* nur sehr wenige "normale" Monozyten das Autoantigen präsentieren und dann auch nur an wenigen MHC II-Komplexen. Es ist Ziel der Behandlung, *in vivo* die "normalen" Monozyten, die das Autoantigen präsentieren und die T-Helfer-Zellen aktivieren, zu verdrängen durch die auf Abschaltung der Antikörperprodukte programmierten, genmanipulierten Monozyten.

Die erfindungsgemäße monoantigene antigen-präsentierende Zelle kann insbesondere eine erhöhte Anzahl von Homing-Rezeptoren, wie CD44, aufzeigen. Eine Überexpression von Homing-Rezeptoren wird der monoantigenen Antigen-präsentierenden Zelle den Weg in die Lymphknoten weisen, wodurch die genmanipulierten APCs sich vermehrt und schneller in Lymphknoten ansammeln. Die Lymphknoten sind der Ort, an dem die allermeisten Reaktionen des anpassungsfähigen Immunsystems hervorgerufen werden. Dort entfalten die genmanipulierten APCs daher eine um ein vielfaches höhere Wirkung als außerhalb der Lymphknoten.

In einer bevorzugten Ausführungsform der erfindungsgemäßen monoantigenen antigen-präsentierenden Zelle bewirkt eine Transformation eine Erhöhung der Anzahl von Homing-Rezeptoren und/oder eine Suppression von Funktionen der B7-, CD40-Rezeptoren.

Insbesondere mit Antisense-Nukleinsäuren kann die B7- und/oder CD40-Rezeptor Expression in der erfindungsgemäßen monoantigenen antigen-präsentierenden Zelle verhindert oder reduziert werden..

Alternativ kann mit Nukleinsäuen eine Unterdrückung der Expression der B7- und/oder CD40-Rezeptoren durch Co-Suppression in der erfindungsgemäßen monoantigene antigen-präsentierende Zelle bewirkt werden.

Die erfindungsgemäße monoantigene antigen-präsentierende Zelle kann Nukleinsäuren enthalten oder transformiert sein, die eine Expression von mit B7- und/oder CD40-Rezeptoren affinen Strukturen aufweisenden Proteinen oder Peptiden bewirken. Damit werden Proteine gebildet, die durch Komplexbildung mit B7 - und/oder CD40-Rezeptoren praktisch neutralisieren. Insbesondere kommen dazu CTLA4, CD28, Antikörper, F(ab)_{2,} scFv und/oder F_{ab}-Fragmente als Proteine in Betracht.

In einer weiteren bevorzugten Ausführungsform enthält die erfindungsgemäße monoantigene antigen-präsentierende Zelle Nukleinsäuren, die für eine Signalsequenz kodieren oder Expressionsprodukte einer Signalsequenz, die den Verbleib der Expressionsprodukte im endoplasmatischen Retikulum, dem Golgi-Apparat, dem Trans-Golgi-Netzwerk oder intrazellulären Vesikeln bewirkt.

Die erfindungsgemäße monoantigene antigen-präsentierende Zelle wird zur Expression von Antigenen mit Nukleinsäuren transformiert, die den Transport der exprimierten Antigene in MHC II-Kompartimente der Zellen ermöglichen.

Alle genmanipulierten Monozyten präsentieren an den meisten ihrer MHC-Komplexe das Autoantigen, während nur sehr wenige "normale" Monozyten überhaupt das Autoantigen präsentieren und dann auch nur an wenigen MHC-Komplexen. Es ist Ziel der Behandlung, die "normalen" Monozyten, die das Autoantigen präsentieren und die T-Helfer-Zellen aktivieren, zu verdrängen durch die auf Abschaltung der Antikörperprodukte programmierten, genmanipulierten Monozyten. Es ist sehr wichtig, das Antigen (die Antigene) auch in einer Form zu verabreichen, die den Transport in die MHC II-Endosomen ermöglicht. Nur so kann zuverlässig eine Präsentation an MHC II erreicht werden. Bei einem gentechnischen Eingriff erfolgt dies in der Regel durch Manipulation des offenen Leserasters, so daß dem Leseraster eine Signalsequenz für dieses Kompartiment vorgeschaltet wird. Eine genetische Manipulation hat den zusätzlichen Vorteil, daß sie eine viel längere Halbwertzeit hat, als z.B. Oligonukleotide oder Peptide. Eine stabile Integration von Genen führt sogar zu einer permanenten Veränderung der Eigenschaft der Zielzellen.

Die entsprechenden Nukleinsäuren können dabei DNA, RNA, Oligonukleotide, Polynukleotide, Ribozyme, Peptidnukleinsäuren (PNA) sein.

Vorzugsweise besitzt die DNA Regulationselemente wie Enhancer, Promotoren, polyA-kodierende 3'-Enden zur Transkription der DNA in RNA, die RNA Regulationselemente zur Translation der RNA in Protein. Die Regulationselemente sorgen für eine effiziente Expression der Gene.

Die Herstellung der erfindungsgemäßen monoantigenen antigen-präsentierenden Zelle erfolgt zum Beispiel durch ex vivo oder in vivo Verfahren. Dabei wird vorzugsweise eine antigen-präsentierende Zelle ex vivo oder in vivo durch Behandlung mit Viren viralen Vektoren, bakteriellen Vektoren, Plasmiden, die durch Elektroporationstechniken, Iontophorese, ballistischen Methoden und/oder anderen Techniken zur Einschleusung von Molekülen in eine monoantigene antigen-präsentierende Zelle transformiert.

In einer weiteren Ausführungsform kann eine antigen-präsentierende Zelle oder eine monoantigene antigen-präsentierende Zelle durch Behandlung mit Viren, viralen Vektoren, bakteriellen Vektoren, Plasmiden, die durch Elektroporationstechniken, Iontophorese, ballistischen Methoden und/oder anderen Techniken zur Einschleusung von Molekülen in eine Zelle mit supprimierter Funktion co-stimulatorischer Rezeptoren transformiert werden oder die Expression co-stimulatorischer Rezeptoren durch Verhinderung von deren Expression oder die co-stimulatorischen Rezeptoren werden durch Reaktion mit affinen Strukturen an einer Stimulation von T-Zellen, die an die monoantigene antigen-präsentierende Zelle gebunden sind, gehindert.

Für die Unterdrückung der Co-Stimulation, die letztendlich ein Unterdrücken der Produktion eines oder mehrerer Proteine oder die Behinderung des Proteins oder der Proteine bedeutet, kommen verschiedene Strategien in Frage:
1. Antisense-Ansatz
2. Co-Suppression
3. Bindung des co-stimulatorischen Moleküls.

- Zu 1.:: In diesem Fall müssen Antisense-Nukleinsäuren in Kontakt mit der mRNA des co-stimulatorischen Moleküls kommen. Sie binden dann wahrscheinlich das Molekül und verhindern die Translation. Als Nukleinsäuren kommen eine große Bandbreite von Molekülen, wie RNAs, DNAs, PNAs, Ribozyme, in Frage. Auch hier würde ein gentechnischer Eingriff für die größte Halbwertszeit des Effekts sorgen.
- Zu 2.:: Es hat sich inzwischen herausgestellt, dass man die Produktion eines Genproduktes auch durch Integration einer möglichst homologen Sense-Gensequenz erreichen kann. Der Mechanismus ist noch völlig unbekannt.
- Zu 3.:: Die Bindung des co-stimulatorischen Moleküls z.B. durch spezifische Antikörper verhindert, daß dieses Molekül Kontakt mit dem avisierten Rezeptor auf einer T-Zelle aufnimmt und verhindert so die Aktivierung der T-Zelle. Die externe Zugabe solcher Bindemoleküle hat den Nachteil, dass sie auf alle Antigen-präsentierenden Zellen wirken und damit jede Immunreaktion verhindern. Um Immunreaktionen spezifisch zu behindern, haben wir ein Modell entworfen, bei dem die co-stimulatorischen Moleküle bereits in der Zelle, in einem intrazellulären Kompartiment gebunden werden. In einer Erweiterung dieses Modells soll dafür gesorgt werden, dass das Bindemolekül im intrazellulären Kompartiment zurückgehalten wird, so dass der co-stimulatorische Rezeptor erst gar nicht zur Plasmamembran gelangt. Hierfür sind Signalsequenzen bekannt, die dem offenen Leseraster des Bindemoleküls zugefügt werden müssen. Dieser intrazelluläre Ansatz lässt sich gut an isolierten Zellen durchführen. Auch hier ist ein gentechnischer Eingriff zu bevorzugen.

Die gewünschten Effekte können auch erreicht werden, wenn nur eines der beiden Ziele mit einem gentechnischen Eingriff vorgenommen wird. In diesem Fall können statt der Gene z.B. folgende anderen Moleküle eingesetzt werden:
1. Behinderung der Co-Stimulation durch
   Nukleinsäuren (zumeist komplementär zur Zielsequenz) bei denen es sich z.B. um Oligonukleotide, Polynukleotide, Ribozyme, Peptidnukleinsäuren (PNAs) handeln kann, Antikörper oder andere Moleküle, die die co-stimulatorischen Moleküle binden.
2. Antigen Kontaktierung durch
   Proteine, Peptide, Peptidomimetica.

Es werden nach dem erfindungsgemäßen Verfahren insbesondere Moleküle wie Antikörper, Proteine, Peptide, Peptidomimetica, CTLA4, CD28, CD40L und/oder Bestandteile und/oder Kombinationen dieser Moleküle, die z.B. B7-1, B7-2, CD40 binden, welche eine in Gegenwart einer Antigenpräsentation stattfindende Co-Stimulation der T-Zelle behindert, mit der monoantigenen antigen-präsentierenden Zelle oder der antigen-präsentierenden Zelle in Kontakt gebracht.

Es kann vorteilhaft sein, die Moleküle mittels Vehikeln, wie Liposomen, Hydrogele, Zyklodextrine, biologisch abbaubare Nanokapseln, bio-adhäsive Mikrokugeln und/oder durch Elektroporationstechniken, Iontophorese, ballistische Methoden und/oder andere Techniken zur Einschleusung von Molekülen in die monoantigene antigen-präsentierende Zelle oder die antigen-präsentierende Zelle zu transferieren.

Nukleinsäuren können insbesondere durch Viren, virale Vektoren, bakterielle Vektoren, Plasmide, die durch Elektroporationstechniken, Iontophorese, ballistische Methoden und/oder andere Techniken zur Einschleusung von Molekülen in die monoantigene antigen-präsentierende Zelle oder die antigen-präsentierende Zelle transferiert werden.

Erfindungsgemäß beansprucht wird ein Arzneimittel, enthaltend mindestens eine erfindungsgemäße monoantigene antigen-präsentierende Zelle. Vorzugsweise ist das erfindungsgemäße Arzneimittel als Infusionslösung zur intravenösen oder intraperitonealen Applikation formuliert. Die Formulierung ist so gewählt, dass bei Verabreichung des Arzneimittels keine wesentliche Beeinträchtigung der Wirksamkeit der erfindungsgemäßen monoantigenen antigen-präsentierenden Zelle erfolgt.

Die erfindungsgemäße monoantigene antigen-präsentierende Zelle kann insbesondere zur Herstellung eines Arzneimittels zur Behandlung von ungewollten Immunreaktionen, wie Autoimmunerkrankungen und Allergien oder gewollt hervorgerufenen Immunreaktionen, wie bei Immunisierungen verwendet werden.

Weiterhin kann die erfindungsgemäße monoantigene antigen-präsentierende Zelle zur Herstellung eines Arzneimittels zur Behandlung von Immunreaktionen gegen allologe und/oder xenologe Gewebsmerkmale verwendet werden.

Erfindungsgemäß beansprucht werden insbesondere Verwendungen, bei denen die zu behandelnden Immunreaktionen in Verbindung mit Antigenen oder deren Gensequenzen und/oder Teilen davon stehen und ausgewählt sind aus der Gruppe bestehend aus
- Enzymen, deren Gensequenzen und/oder Teilsequenzen, insbesondere Glutamic acid decarboxylase (GAD), Rezeptor-Typ Protein Tyrosin Phosphatase IA-2Beta, Antigen: H⁺K⁺ATPase, U1RNP, Transglutaminase, Argininosuccinatlyase (ASL), Tyrosinase-related protein-2, Thyroid Peroxidase, Faktor VIII, Faktor IX;
- Rezeptoren, deren Gensequenzen und/oder Teilsequenzen, insbesondere Acetylcholinrezeptor vom Nicotintyp, β1-adrenerger-Rezeptor, α1-adrenerger-Rezeptor, Angiotensin-2-AT1-Rezeptor, Glutamat-Rezeptor, Thyrotropin-stimulierendes Hormon (TSH)-Rezeptor, LFA-1, HLA-B27, Epididymal Protein DE, Zona Pellucida (ZP)-3 Glycoprotein, Zona Pellucida (ZP)-4 Glycoprotein, Follicle-Stimulating Hormone (FSH) Rezeptor, Sperm Immunogen SP-10 oder Sperm Protein SP-10;
- Hormone oder Botenstoffe, deren Gensequenzen und/oder Teilsequenzen, insbesondere Insulin, Thyroglobulin, Follicle-Stimulating Hormone (FSH), Prostaglandin F2 alpha, Gonadotropin-Releasing Hormone (GnRH), Oestradiol-17beta, Oestrogen, Luteinizing Hormon (LH) Rezeptor, Inhibin, Testosteron, Androgen, Chorionic Gonadotrophin (CG), Interleukine, Interferone, Cytokine, Chemokine, Bone Morphogenetic Factors, β-Interferon, Estradiol;
- Strukturproteine, deren Gensequenzen und/oder Teilsequenzen, insbesondere Myelin Basic Protein (MBP), Proteolipid protein (PLP), Myelin oligodendrocyte glycoprotein (MOG), α-Fodrin, Nicht-erythroides α-Spectrin, Beta-Amyloid Precursor Protein (beta-APP), Typ 2 Kollagen, Sperm Plasma Membran Protein PH-20;
- Antigene, deren Gensequenzen und/oder Teilsequenzen, insbesondere CENP-A Autoantigen, Beta2GP-I, ribosomales P Protein, Ro/SSA, La/SSB, Sm/RNP, Sm, Scl-70, Jo-1, BCOADC-E2, Albumin, Glucagon, Inselzellantigene, Retinal S Ag;
- Allergene, die eine IgE-Antwort auslösen, deren Gensequenzen und/oder Teilsequenzen, insbesondere Der f1, Der f2, Der f3, Der p 1, Der p 2, Der p 3, Der p 4, Der p 5, Der p 8, Eur m 1, Lep d 2, Fel d 1, Can f1, Can f2, Mus m 1, Rat n 1, Bla g 1, Bla g 2, Bla g 4, Bla g 5, Per a 1, Bienengift Phospholipase A2 (PLA2), Group V major allergen Phl p 5b von Timothy Grass Pollen, Hom s 1.

Weiterhin wird erfindungsgemäß beansprucht eine Verwendung bei der die zu behandelnden Immunreaktionen in Verbindung mit allologen und/oder xenologen Gewebsmerkmalen, deren Gensequenzen und/oder Teilsequenzen, insbesondere MHC I, MHC II, Rhesus Faktor stehen.

### Entwicklung einer Gentherapie

Das hier vorgestellte Verfahren zur Reduzierung von Immunantworten z.B. zur Behandlung von Autoimmunerkrankungen mit klar definiertem Autoantikörperprofil basiert auf der gentechnischen Manipulation von bestimmten Blutzellen vorzugsweise außerhalb des Körpers, die dann anschließend wieder in den Organismus zurückgeführt werden. Ziel dieser Behandlung ist unter anderem das spezifische Abschalten einer chronischen, durch das Immunsystem getriebenen Produktion von Autoantikörpern, welche die Krankheit auslösen.

### Anwendungsgebiete der Gentherapie

Das Verfahren zur spezifischen Abschaltung von Immunreaktionen ist immer dort sinnvoll, wo ein oder mehrere molekular definierte Targets (Antigene, Autoantigene) bekannt sind. Diese können beispielsweise mit einer Autoimmunerkrankung oder Allergie assoziiert seien.

Z.B. handelt es sich dabei um Krankheiten mit Autoantikörper-vermittelten Autoimmunreaktionen, d.h. um Krankheiten, bei denen die "Bindungsstrukturen" dieser Autoantikörper (Autoantigene, Targets) bekannt sind und pathogenetische Bedeutung haben.

Beispiele für Krankheiten mit bekannten, die Autoantikörper-bindenden molekularen Strukturen (Epitope) sind:
- Myasthenia gravis (Autoantikörper gegen den Acetylcholinrezeptor)
- Morbus Basedow (Autoantikörper gegen den TSH-Rezeptor der Schilddrüse)
- Dilatative Cardiomyopathie (DCM, Autoantikörper gegen den β1-adrenergen Rezeptor der Herzmuskelzellen)
- bestimmte Formen des insulinabhängigen Diabetes (Autoantikörper gegen Insulin)
- bestimmte Formen des malignen Bluthochdrucks (Autoantikörper gegen den Angiotensin- und/oder α1-adrenerger-Rezeptor.

### Behandlung mit genmanipulierten patienteneigenen Blutzellen: Prinzip der vorgeschlagenen Gentherapie

Innerhalb des Immunsystems spielen die Antikörper als Abwehrmoleküle ("humorale Immunreaktion") eine wichtige Rolle. Sie werden von ausgereiften B-Lymphozyten produziert. Die Induktion und Produktion der Antikörper erfolgt allerdings nicht losgelöst von den restlichen Zellen des Immunsystems; vielmehr wird diese humorale Immunreaktion von anderen Zellen des Immunsystems gesteuert. Die Immunreaktion lässt sich nicht aufrechterhalten ohne die Hilfe und Vermittlung von Antigen-präsentierenden Zellen und T-Helfer-Lymphozyten. Die molekularen Mechanismen der Interaktion - des Zell-Zell-Kontakts - von Antigen-präsentierenden Zellen mit den T-Helfer-Lymphozyten ist auf Rezeptorebene bis ins Detail bekannt.

Neben dem zu präsentierenden Antigen sind auf der Zelloberfläche verschiedene Rezeptoren und Hilfsrezeptoren am Zell-Zell-Kontakt und der Zellaktivierung beteiligt. Ein essentielles Molekül der interzellulären Wechselwirkung bei der Antigenpräsentation (Kontakt von Antigen-präsentierenden Zellen mit den T-Helfer-Lymphozyten) sind costimulierende Rezeptoren mit den Bezeichnungen B7-1 und B7-2. Auch CD40 spielt bei dieser Signaltransduktion eine Rolle.

Die Funktionen von B7 und CD40 sind Inhalt zahlreicher Publikationen (Daikh *et al.* 1997; Greenfield *et al.* 1998; Johnson-Leger *et al.* 1998; McAdam *et al.* 1998; Vyth-Dreese *et al.* 1995) und werden auch umfangreich in Lehrbüchern abgehandelt (s. z.B. Janeway and Travers 1997).

Das konzipierte Verfahren beruht auf zwei parallelen Eingriffen an patienteneigenen Antigen-präsentierenden Zellen. Die Eingriffe erfolgen mittels geeigneter Sonden und bewirken
- die Abschaltung von B7 und damit die Verhinderung der Ausbildung des Moleküls auf der Oberfläche der Antigen-präsentierenden Zellen und/oder die Abschaltung von CD40 und
- gleichzeitig eine starke Präsentation des Autoantigens auf den Antigen-präsentierenden Zellen.

Sollte die Manipulation der Zellen, die z.B. Monozyten sind, *in vitro* erfolgen, werden die Zellen in die Blutbahn des Spenders zurückgegeben. Die genmanipulierten Monozyten schalten nunmehr die im Organismus befindlichen entsprechenden T-Helfer-Lymphozyten ab. Die genmanipulierten Zellen treten dabei unmittelbar in Konkurrenz zu den bereits im Organismus (vorzugsweise Blutbahn und Lymphsystem) vorhandenen Autoantigen-präsentierenden Monozyten, die allerdings B7 auf der Zelloberfläche tragen und normalerweise die T-Helfer-Lymphozyten und damit unter anderem die Antikörperproduktion aktivieren.

### Behandlung von Autoimmunerkrankungen, Allergien und Transplantationen: Stand der Technik

Kausaltherapien zur Behandlung von Autoimmunerkrankungen und Allergien existieren nicht. Von wenigen Ausnahmen abgesehen (extrakorporale Elimination der Antikörper aus dem Blut der Patienten bzw. Plasmapherese), erfolgt die Behandlung von Autoimmunerkrankungen und Allergien medikamentös durch eine Hemmung von Reaktionen des Immunsystems.

Nach wie vor am gebräuchlichsten ist die medikamentöse Behandlung von Autoimmunerkrankungen, Allergien und Transplantationen mit immunsuppressiven Präparaten wie Cortison und dessen Abkömmlingen, Cyclosporin, Beta-Interferon oder Zytostatika (Methothrexat). Desweiteren werden Antikörper, Antagonisten und Oligonukleotide gegen verschiedene Signalkomponenten des Immunsystems mit dem Zweck erprobt, die Aktivierung von T-Zellen zu verhindern. Solche Arzneimittel sind bestenfalls selektiv, nie aber spezifisch gegen die falsch programmierten Autoimmunreaktionen gerichtet. Immunsuppressiva haben daher auf wichtige, notwendige und nützliche Teile des Immunsystems eine negative Wirkung; aus diesem Grund und ihrer Nebenwirkungen wegen ist ihr Einsatz begrenzt.

Von den immunsuppressiven Therapien unterscheidet sich das vorgelegte Konzept einer Gentherapie zur Reduktion von spezifischen Immunreaktionen grundlegend. Es wird eine spezifische Abschaltung fehlgeleiteter immunologischer Reaktionen durch gentechnisch umprogrammierte patienteneigene Blutzellen durchgeführt.

Die aufgezeigte Methodik ist als allgemein gültiges Konzept zur Reduzierung von Immunantworten gedacht. Es sollte mit diesem Grundkonzept möglich sein jede Immunreaktion des anpassungsfähigen Immunsystems wieder abzustellen. Außerdem können nach belieben solche Immunreaktionen hervorgerufen und dann wieder abgestellt werden.

### Literatur

**Daikh D., Wofsy D. and Imboden J.** (1997) The CD28-B7 costimulatory pathway and its role in autoimmune disease. *JLeukoc Biol* **62**, 156-62.

**Greenfield E., Nguyen K. and Kuchroo V.** (1998) CD28/B7 costimulation: a review. *Crit Rev Immunol* **18**, 389-418.

**Janeway J.C.A. and Travers P.** (1997) Immunobiology - The immune system in health and disease. Current Biology Ltd./Garland Publishing Inc., London, San Francisco and New York

**Johnson-Leger C., Christensen J. and Klaus G**. (1998) CD28 co-stimulation stabilizes the expression ofthe CD40 ligand on T cells. *Int Immunol* **10**, 1083-91.

**McAdam A., Schweitzer A. and Sharpe A.** (1998) The role of B7 co-stimulation in activation and differentiation of CD4+ and CD8+ T cells. *Immunol Rev* **165**, 231-47.

**Steinman L.** (1994) Autoimmunerkrankungen. *Spektrum der Wissenschaften (Spezial: Das Immunsystem)* 64-73.

**Vyth-Dreese F., Dellemijn T., Majoor D. and de Jong D.** (1995) Localization in situ of the co-stimulatory molecules B7.1, B7.2, CD40 and their ligands in normal human lymphoid tissue. *Eur J Immunol* **25**, 3023-9.

## Patentansprüche

1. Antigen präsentierende Zelle, die überwiegend vorher bestimmte Antigene präsentiert (monoantigene antigen-präsentierende Zelle) dadurch gekennzeichnet, dass in der monoantigenen antigen-präsentierenden Zelle eine der Funktionen co-stimulatorischer Rezeptoren, wie ein B7- und/oder CD40-Rezeptor supprimiert ist.

2. Monoantigene antigen-präsentierende Zelle nach Anspruch 1, dadurch gekennzeichnet, dass die monoantigene antigen-präsentierende Zelle ein Monozyt, eine dendritische Zelle und/oder ein Makrophage ist.

3. Monoantigene antigen-präsentierende Zelle nach Anspruch 1 und/oder 2, dadurch gekennzeichnet, dass durch Transformation einer antigen-präsentierenden Zelle mit nukleinsäurehaltigem Material eine erhöhte Expression eines Antigens erfolgt und die antigen-präsentierende Zelle im wesentlichen nur eine Sorte eines Antigens präsentiert
und/oder
die monoantigene antigen-präsentierende Zelle eine erhöhte Anzahl von Homing-Rezeptoren, wie CD44, aufweist
und/oder
eine Transformation eine Suppression von Funktionen der B7-, CD40-Rezeptoren und/oder eine Erhöhung der Anzahl von Homing-Rezeptoren bewirkt.

4. Monoantigene antigen-präsentierende Zelle nach mindestens einem der Ansprüche 1 bis 3 enthaltend Antisense-Nukleinsäuren zur Verhinderung der B7- und/oder CD40-Rezeptor Expression
und/oder
enthaltend Nukleinsäuren, die eine Unterdrückung der Expression der B7-und/oder CD40-Rezeptoren durch Co-Suppression bewirken
und/oder
enthaltend Nukleinsäuren, die eine Expression von mit B7- und/oder CD40-Rezeptoren affinen Strukturen aufweisenden Proteinen oder Peptiden bewirken.

5. Monoantigene antigen-präsentierende Zelle nach Anspruch 4, wobei die Proteine, die affine Strukturen zu B7-Rezeptoren aufweisen CTLA4, CD28, Antikörper, F(ab)_{2,} scFv und/oder F_{ab}-Fragmente sind.

6. Monoantigene antigen-präsentierende Zelle nach Anspruch 4 und/oder 5, wobei die Nukleinsäuren für eine Signalsequenz kodieren oder die Expressionsprodukte eine Signalsequenz besitzen, die den Verbleib der Expressionsprodukte im endoplasmatischen Retikulum, dem Golgi-Apparat, dem Trans-Golgi-Netzwerk oder intrazellulären Vesikeln bewirkt.

7. Monoantigene antigen-präsentierende Zelle nach mindestens einem der Ansprüche 3 bis 6, dadurch gekennzeichnet, dass die monoantigene antigen-präsentierende Zelle zur Expression von Antigenen mit Nukleinsäuren transformiert ist, die den Transport der exprimierten Antigene in MHC II-Kompartimente der Zellen ermöglicht.

8. Monoantigene antigen-präsentierende Zelle nach Anspruch 7, dadurch gekennzeichnet, dass die DNA Regulationselemente wie Enhancer, Promotoren, polyA-kodierende 3'-Enden zur Transkription der DNA in RNA enthält, die RNA Regulationselemente zur Translation der RNA in Protein enthält.

9. Verfahren zur Herstellung der monoantigenen antigen-präsentierenden Zelle nach mindestens einem der Ansprüche 1 bis 8 durch ex vivo oder in vivo Verfahren.

10. Verfahren nach Anspruch 9, wobei Moleküle wie Antikörper, Proteine, Peptide, Peptidomimetica, CTLA4, CD28, CD40L und/oder Bestandteile und/oder Kombinationen dieser Moleküle, die z.B. B7-1, B7-2, CD40 binden, welche eine in Gegenwart einer Antigenpräsentation stattfindende Co-Stimulation der T-Zelle behindert, mit der monoantigenen antigen-präsentierenden Zelle oder der antigen-präsentierenden Zelle in Kontakt gebracht werden.

11. Arzneimittel enthaltend mindestens eine monoantigene antigen-präsentierende Zelle nach mindestens einem der Ansprüche 1 bis 8.

12. Verwendung mindestens einer monoantigenen antigen-präsentierenden Zelle nach mindestens einem der Ansprüche 1 bis 8 zur Herstellung eines Arzneimittels zur Behandlung von ungewollten Immunreaktionen, wie Autoimmunerkrankungen und Allergien oder gewollt hervorgerufenen Immunreaktionen, wie bei Immunisierungen
und/oder
zur Herstellung eines Arzneimittels zur Behandlung von Immunreaktionen gegen allologe und/oder xenologe Gewebsmerkmale.

13. Verwendung nach Anspruch 12, wobei die zu behandelnden Immunreaktionen in Verbindung mit Antigenen oder deren Gensequenzen und/oder Teilen davon stehen ausgewählt aus der Gruppe bestehend aus
- Enzymen, deren Gensequenzen und/oder Teilsequenzen, insbesondere Glutamic acid decarboxylase (GAD), Rezeptor-Typ Protein Tyrosin Phosphatase IA-2Beta, H⁺K⁺ATPase, U1RNP, Transglutaminase, Argininosuccinatelyase (ASL), Tyrosinase-related protein-2, Thyroid Peroxidase, Faktor VIII, Faktor IX;
- Rezeptoren, deren Gensequenzen und/oder Teilsequenzen, insbesondere Acetylcholinrezeptor vom Nicotintyp, Myasthenia gravis, β1-adrenerger Rezeptor, α1-adrenerger-Rezeptor, Angiotensin-2-AT1-Rezeptor, Glutamat-Rezeptor, Thyrotropin-stimulierendes Hormon (TSH)-Rezeptor, LFA-1, HLA-B27, Epididymal Protein DE, Zona Pellucida (ZP)-3 Glycoprotein, Zona Pellucida (ZP)-4 Glycoprotein, Follicle-Stimulating Hormone (FSH) Rezeptor, Sperm Immunogen SP-10 oder Sperm Protein SP-10;
- Hormone oder Botenstoffe, deren Gensequenzen und/oder Teilsequenzen, insbesondere Insulin, Thyroglobulin, Follicle-Stimulating Hormone (FSH), Prostaglandin F2 alpha, Gonadotropin-Releasing Hormone (GnRH), Oestradiol-17beta, Oestrogen, Luteinizing Hormon (LH) Rezeptor, Inhibin, Testosteron, Androgen, Chorionic Gonadotrophin (CG), Interleukine, Interferone, Cytokine, Chemokine, Bone Morphogenetic Factors, β-Interferon, Estradiol;
- Strukturproteine, deren Gensequenzen und/oder Teilsequenzen, insbesondere Myelin Basic Protein (MBP), Proteolipid protein (PLP), Myelin oligodendrocyte glycoprotein (MOG), α-Fodrin, Nicht-erythroides α-Spectrin, Beta-Amyloid Precursor Protein (beta-APP), Typ 2 Kollagen, Sperm Plasma Membran Protein PH-20;
- Antigene, deren Gensequenzen und/oder Teilsequenzen, insbesondere CENP-A Autoantigen, Beta2GP-I, ribosomales P Protein, Ro/SSA, La/SSB, Sm/RNP, Sm, Scl-70, Jo-1, BCOADC-E2, Albumin, Glucagon, Inselzellantigene, Retinal S Ag;
- Allergene, die eine IgE-Antwort auslösen, deren Gensequenzen und/oder Teilsequenzen, insbesondere Der f1, Der f2, Der f3, Der p 1, Der p 2, Der p 3, Der p 4, Der p 5, Der p 8, Eur m 1, Lep d 2, Fel d 1, Can f1, Can f 2, Mus m 1, Rat n 1, Bla g 1, Bla g 2, Bla g 4, Bla g 5, Per a 1, Bienengift Phospholipase A2 (PLA2), Group V major allergen Phl p 5b von Timothy Grass Pollen, Hom s 1
und/oder
in Verbindung mit allologen und/oder xenologen Gewebsmerkmalen, deren Gensequenzen und/oder Teilsequenzen, insbesondere MHC I, MHC II, Rhesus Faktor steht.
